# EUROPEAN PATENT SPECIFICATION

(11) **EP 4 497 425 B1**
(45) Date of publication and mention of the grant of the patent: **01.04.2026**
(21) Application number: 23187487.6
(22) Date of filing: 25.07.2023
(51) Int. Cl.: A61F 13/42, A61F 13/84

(54) **MONITORING DEVICE FOR TESTING CONDUCTIVE PARTS ON ABSORBENT ARTICLES AND METHODS THEREOF**
ÜBERWACHUNGSVORRICHTUNG ZUM TESTEN LEITFÄHIGER TEILE AUF SAUGFÄHIGEN ARTIKELN UND VERFAHREN DAFÜR
DISPOSITIF DE SURVEILLANCE POUR TESTER DES PARTIES CONDUCTRICES SUR DES ARTICLES ABSORBANTS ET PROCÉDÉS ASSOCIÉS

(43) Date of publication of application: 29.01.2025
(73) Proprietor: Ontex BV, 9255 Buggenhout (BE); Ontex Group NV, 9320 Erembodegem (BE)
(72) Inventor: HELLMOLD, Jens, 59269 Beckum (DE); PEPERMANS, Manu, 2018 Antwerpen (BE); MADAAN, Ankit, 9000 Ghent (BE)
(74) Representative: Macchetta, Andrea

(56) References cited:
- US-A1- 2022 233 363
- US-A1- 2023 129 630
- US-A1- 2023 165 729

## Description

### TECHNICAL FIELD

The present disclosure is directed to a monitoring device for testing conductive elements of exudate detection sensors contained by an absorbent article, generally the absorbent article being a disposable personal hygiene article selected from pants, diapers and pads for incontinence. The incontinence disposable personal hygiene articles such as pants, diapers and/or pads typically being for adults i.e. adult pants, adult diapers and/or adult pads. The disclosure further relates to methods for for testing one or more conductivity parameters of such sensors and particularly in a production facility for quality control of such articles prior commercialisation thereof.

### BACKGROUND

Absorbent articles for personal hygiene are designed to absorb and contain bodily exudates, such as a large quantity of urine. Non-limiting examples of disposable absorbent articles include diapers, pants, training pants, pads, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP2496197B1, EP2739254B1, and EP2582341B1) directed to sensors to sense a condition such as temperature from body or moisture from incontinence. The sensor comprises a signal processing unit, a transmitter and a power supply, typically in form of a battery. These elements are arranged on a flexible substrate in low profile enabling disposition adjacent to the human body. A complex series of mathematical and statistical manipulations are then needed in order to determine wetness events and wetness levels.

While such devices allow monitoring conditions of the human body and can also be used as a moisture sensor, it represents also relatively costly solution. It would not be seen appropriate to dispose of the sensor together with a (disposable) absorbent article. If the sensor, however, is to be reused, the sensing area has potentially been exposed to moisture. Therefore this concept does not allow for simple usage.

Examples of articles that provide improvements to the above drawbacks are disclosed in EP3415130, WO/2018/228822, WO/2018/229017, EP3461257, and EP3451988. Further prior art is known from US2023/165729.

Yet there remains a need for apparatuses and methods that may reliably aid quality assurance of sensors comprised in such absorbent articles by determining correct functioning prior to usage, for example within a manufacturing plant, preferably such devices and methods allowing for simultaneous testing and/or verification of a plurality of such sensors and/or articles.

### SUMMARY

In a first aspect the disclosure relates to a testing device for testing one or more conductivity parameters of a sensor-comprising absorbent article, said device comprising: a computing device, preferably being a hand-held computing device, comprising a processor and an input receiver; a clip-on device comprising a power source, transmitter, a processing unit, and one or more electrical terminals in electrical communication with at least one of the transmitter and the processing unit; wherein said terminals are adapted to come into contact with corresponding connection tracks of a sensor, and wherein an absorbent article comprises said sensor; said computing device and said clip-on device are distinct components in data communication with each other and wherein a plurality of conductivity parameters are acquired by the clip-on device and transmitted by the transmitter to the computing device, wherein the acquired plurality of conductivity parameters are further processed by the processor and/or processing unit to provide an indication to a user related to the proper functioning of the sensor preferably via a user interface comprised by said computing device and in data communication with said display.

In a second aspect the disclosure relates to a method for testing one or more conductivity parameters of a sensor-comprising absorbent article, said method comprising the steps of: providing a testing device comprising a computing device, preferably a hand-held computing device such as a tablet, comprising a processor and an input receiver; and a clip-on device comprising a power source, transmitter, a processing unit, and one or more electrical terminals in electrical communication with at least one of the transmitter and the processing unit; providing an absorbent article comprising a sensor for exudate detection, said sensor comprising a pattern of electrically conductive material and a plurality of connection tracks; joining the clip-on device to the absorbent article such that at least some of the one or more electrical terminals is in electrical communication corresponding one or more connection tracks; acquiring one or more analogue conductivity parameters and converting said analogue conductivity parameters to a digital format comprising a binary signal(s); transmitting said binary signal(s) from the clip-on device to the computing device; and processing said signal(s) to automatically provide a status warning to a user in relation to the proper functioning of the sensor, wherein the processing step comprises comparing each said signal with a predetermined threshold.

### BRIEF DESCRIPTION OF THE FIGURES

**FIG. 1** Is a perspective view of a testing device according to an embodiment of the disclosure.
**FIG. 2** Is a perspective view of a clip-on device for a testing device according to an embodiment of the disclosure.
**FIG. 3** Is a planar view of an absorbent article comprising a sensor for use in a testing device according to an embodiment of the present disclosure.
**FIG. 4** Is a perspective view of a test bench for a testing device according to an embodiment of the disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The absorbent articles of the disclosure will be further illustrated in the below description and in the Figures, though all embodiments described herein may equally be applied onto absorbent articles in the form of pants (or even in the form of feminine hygiene articles such as menstrual pants and/or slips/panties). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

The terms "joined" or "associated" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined above and glue enclosed within the core wrap.

### TESTING DEVICE

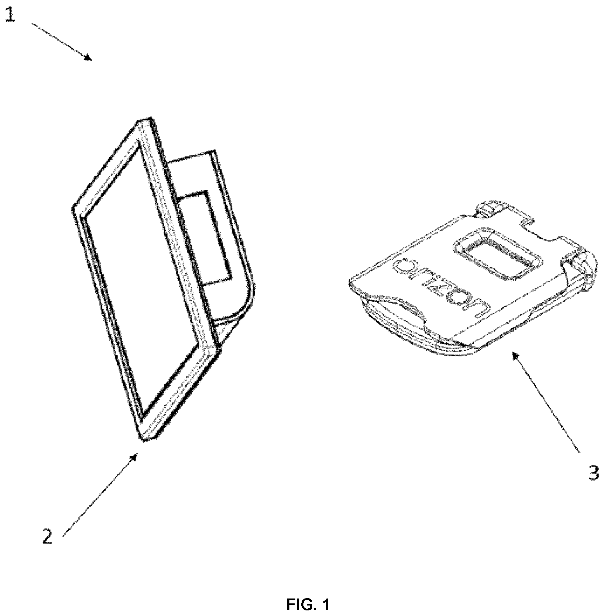

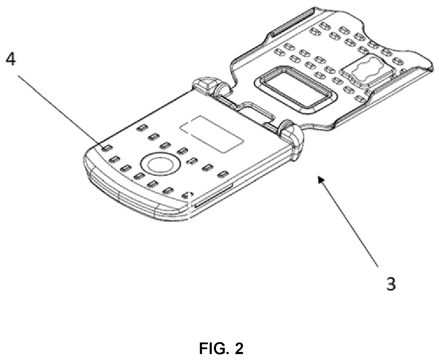

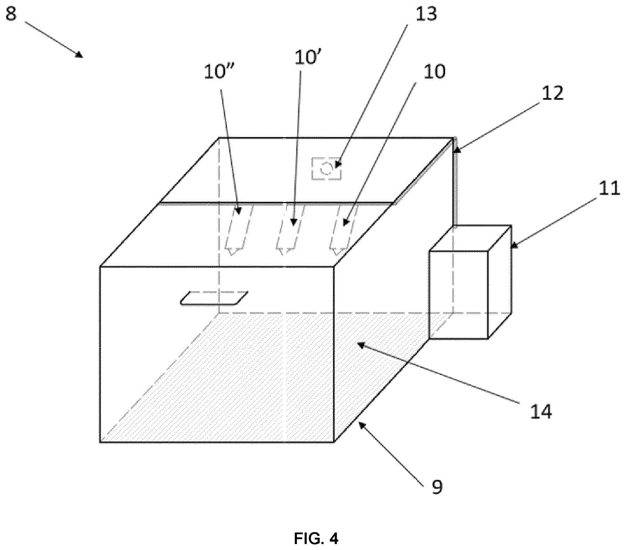

As exemplified in Fig. 1 to Fig. 4, a testing device (1) for testing one or more conductivity parameters of a sensor-comprising absorbent article, comprises: a computing device (2), preferably a hand-held computing device, comprising a processor and an input receiver, and preferably a display; a clip-on device (3) comprising a power source, transmitter (the transmitter may herein be a wireless transmitter but equally encompasses simple transmission by a wire or cable) , a processing unit, and one or more electrical terminals (4) in electrical communication with at least one of the transmitter and the processing unit; wherein said terminals (4) are adapted to come into contact with corresponding connection tracks (5) of a sensor (6), and wherein an absorbent article (7) comprises said sensor (6); wherein the computing device (2) and the clip-on device (3) are distinct components in data communication with each other and wherein when a plurality of conductivity parameters are acquired by the clip-on device (3) and transmitted by the transmitter to the computing device (2), the acquired plurality of conductivity parameters are able to be further processed by the processor and/or processing unit to provide an indication to a user related to the proper functioning of the sensor (6) preferably via a user interface comprised by said computing device (2) and in data communication with said display. Advantageously such a testing device allows for flexibility when testing absorbent articles in a production environment for asserting correct functioning prior to commercialisation, within quality labs, or alternatively within a nursing home or hospital environment for asserting correct functioning for example during initial setting up or when a new batch of articles is delivered.

The testing device is generally adapted to automatically detect and verify product/article type as will be described herein and/or correct clip-on positioning onto the absorbent article. This information may be directly measured by the device or indirectly determined by measuring the one or more conductivity parameters.

The computing device (2) and the clip-on device (3) may alternatively be comprised by the same device, for example the clip-on device may comprise the computing device. Although a user would no longer have the convenience of easily monitoring via for example a separate hand-held computing device, it may be done directly by viewing information from the clip-on e.g. a screen thereof.

The one or more conductivity parameters herein may not only relate to determining the ability of the sensor to detect the presence of exudates but may further relate to determining the proper connection of the clip-on device to the absorbent article. By measuring conductivity parameters relating to appropriate connection, indirect information about appropriate pocket formation for receiving the clip-on device may be collected.

The data communication between the computing device (2) and the clip-on device (3) may be a wire or cable communication for example using a debug probe such as J-Link.

The data communication between the computing device (2) and the clip-on device (3) is preferably wireless. The wireless communication may comprise one or more of Bluetooth, Wi-fi, NFC, Zigbee, Z-Wave, and RFID. Preferably the wireless communication technology used employs radio waves and comprises frequency-hopping spread spectrum technique for secure and reliable communication. Preferably the computing device and the clip-on device are in Bluetooth wireless data communication.

The hand-held device may be selected from the group consisting of a tablet, smartphone, portable computer, and combinations thereof.

The one or more conductivity parameters are typically acquired in analogue format and converted into digital format comprising a binary signal by the processing unit of the clip-on device (3) and transmitted by the transmitter to the computing device (2), wherein the binary signal(s) received by the receiver of the computing device (2) is further processed by the processor to provide an indication to a user related to the proper functioning of the sensor (6) via a user interface comprised by said computing device (2) and in data communication with said display.

The absorbent article (7) herein may be any absorbent article for personal hygiene such as diapers, pants and pads, and the like, that may be disposable; preferably the absorbent article is an incontinence article selected from the group consisting of a diaper, a pant, a pad, and combinations thereof. Preferably, incontinence disposable personal hygiene articles such as pants, diapers and/or pads being for adults i.e. adult pants, adult diapers and/or adult pads.

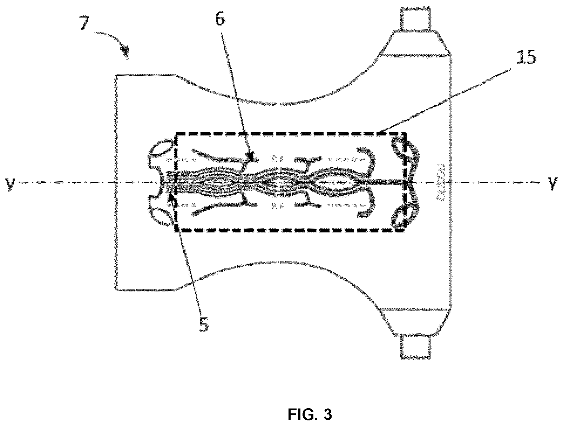

As exemplified in Fig. 3, the absorbent article (7) comprises a sensor (6) that may be applied onto a backsheet of said article. The article typically being constructed to comprise a liquid impermeable topsheet, a substantially liquid impermeable backsheet and an absorbent core (15) positioned between the topsheet and backsheet. The sensor (6) may thus be located and/or positioned on a skin facing surface of the backsheet so that at least a portion of said sensor (6) is exposed to contact with exudates upon a voiding event. The sensor (6) generally being interposed directly and/or indirectly between the absorbent core and the backsheet.

The sensor (6) comprises an electrically conductive material, preferably an electrically conductive ink, having a predetermined pattern. The pattern arranged to sense the presence of exudates in different locations of the absorbent article in an x-y cartesian coordinate system, typically by a change in one or more conductivity parameters such as change in resistance and/or capacitance.

The sensor (6) comprises a plurality of connection tracks (5) wherein more than two, preferably at least 3, even more preferably from 4 to 25, of the said plurality of connection tracks (5) may be in electrical communication with a sensing pattern (or sensing tracks) comprising a plurality of open electrical circuits disposed on a portion of the article (7), preferably the backsheet thereof, and arranged such that each said circuit is able to change one or more conductivity parameters when contacted with exudates and each said circuit being representative of one or more locations of a plurality of locations of an absorbent article in an x-y cartesian coordinate system. The plurality of connection tracks (5) are preferably at least 3, preferably at least 4, more preferably from 5 to 35, connection tracks (5). Examples of suitable sensor-comprising absorbent articles for testing herein are described in EP3760104. When testing the sensor exudates may be simulated by applying a liquid as described herein typically in the form of a saline solution.

The computing device (2) preferably comprises a user interface able to guide step by step the operator through an entire test procedure such as identification of the product and based thereon advise the operator on amount and location that liquid should be applied onto the absorbent article; and automatically display the results of the measurements by relaying a message on whether the test conditions are met or not by the tested product.

In a preferred embodiment, the testing device comprises a test bench (8) comprising a housing (9) for accommodating the absorbent article therein and one or more liquid dispensers (10, 10', 10") arranged to apply a predetermined amount of liquid onto a body facing surface of said absorbent article. Preferably, a plurality of the liquid dispensers (10, 10', 10") are positioned at a distance from one another, preferably such that each dispenser is able to apply a predetermined amount of liquid at one or more of: a different location on the same absorbent article and the same location of a plurality of absorbent articles. Advantageously, a plurality of articles may thus be tested substantially simultaneously.

Preferably, each of the plurality of liquid dispensers comprises at least a first dispenser (10) and at least a second dispenser (10'), wherein the at least first dispenser is adapted to apply a first predetermined amount of liquid to a first absorbent article and the at least second dispenser is adapted to apply a second predetermined amount of liquid to a second absorbent article, wherein the at least first and second absorbent articles have a product characteristic that is the same or different and wherein the at least first and second predetermined amounts of liquid are the same or different respectively, wherein said product characteristic comprises at least one of size, absorbency, and article type. Advantageously, a plurality of articles having e.g. different size and/or absorbency may be tested substantially simultaneously.

The testing device may further comprise a liquid reservoir (11) being in fluid communication with the one or more liquid dispensers (10, 10', 10"), preferably via a liquid impermeable tube or pipe (12).

Preferably, the testing device comprises a camera (13) for taking one or more images of the article at different time intervals before, during and after application of liquid by the one or more liquid dispensers (10, 10', 10"). Images taken by the camera may thus be collected and the acquired one or more conductivity parameters may be clustered with corresponding images that have been processed by imaging analysis and further combined to correlate the said conductivity parameter value to the corresponding optical result extrapolated from the corresponding image. Advantageously, this allows an operator to inspect a plurality of articles substantially remotely yet having the ability to combine visual inputs for each at a later time if/when desired.

In an embodiment, the housing comprises a control chamber (14) adapted for receiving the absorbent article to be tested, the chamber being temperature and/or humidity controlled to room conditions at about 21°C and about 50%RH, preferably the chamber being accessible via an opening that may be sealed by a door.

### METHOD

Methods herein are for testing one or more conductivity parameters of a sensor-comprising absorbent article, and comprise the steps of: providing a testing device (1) comprising a computing device (2), preferably a hand-held computing device, comprising a processor and an input receiver, and preferably a display; and a clip-on device (3) comprising a power source, transmitter, a processing unit, and one or more electrical terminals (4) in electrical communication with at least one of the transmitter and the processing unit; providing an absorbent article (7) comprising a sensor (6) for exudate detection, said sensor comprising a pattern of electrically conductive material and a plurality of connection tracks (5); joining the clip-on device to the absorbent article such that at least some of the one or more electrical terminals (4) is in electrical communication corresponding one or more connection tracks (5); acquiring one or more analogue conductivity parameters and converting said analogue conductivity parameters to a digital format comprising a binary signal(s); transmitting said binary signal(s) from the clip-on device (3) to the computing device (2); and processing said signal(s) to automatically provide a status warning to a user in relation to the proper functioning of the sensor (6), wherein the processing step comprises comparing each said signal with a predetermined threshold. Advantageously this method may enable quick and effective conductivity measurements to be carried out for testing correct functioning of sensor-comprising absorbent articles.

Although less preferable, analogue conductivity parameters may be captured by a detection means such as a multimeter. The computing device may receive analogue measurements that are converted into useful information via a user interface.

Preferably, prior to the step of processing said signal(s) to automatically provide a status warning and/or acquiring one or more analogue conductivity parameters; a predetermined amount of liquid is applied to said article and preferably wherein said predetermined amount of liquid is different for each different product characteristic of said article said product characteristic comprising at least one of size, absorbency, and article type, preferably wherein the article type is selected from a pant, a diaper or a pad. Advantageously this permits to simulate in-use behaviour and test that the circuits are recognising the present of liquid.

The article may be positioned in a flat open state having the most body-facing surface thereof (e.g. the topsheet) facing upwards, preferably so that upon application of a liquid, said surface is the first to come into contact therewith.

A liquid may be applied to the article as described in more detail herein. The liquid may be applied such that different circuits of a plurality of circuits of the sensor and corresponding to different locations of the absorbent article are activated by causing a change in one or more conductivity parameters, and wherein said liquid is applied such that a predetermined number of said circuits are activated corresponding to a liquid application region and/or amount of said liquid, the method comprising the step of verifying automatically that the said circuits are correctly being activated.

The liquid may be a saline solution containing a dye or other component that allows for visual identification of the liquid and its position as it is being absorbed by the absorbent article, such as methylthioninium chloride.

In an embodiment, liquid is applied at one or more locations of the absorbent article and an image capturing means e.g. a camera as described herein above may be used to record visual information data relating to the liquid position after one or more insults and after one or more time intervals. Visual information data may be further combined with the one or more conductivity parameters to automatically provide a status warning to a user in relation to the proper functioning of the sensor (6), for example automatic verification that the one or more circuits of a plurality of circuits corresponding to different locations on the absorbent article are correctly being activated. The comparing step may comprise the step of identifying the circuits responsible for detecting voiding events corresponding to an area of the article comprising a liquid according to the visual information data to provide a first circuit identification cluster; identifying the circuits that have been activated according to the tested one or more conductivity parameters to provide a second circuit identification cluster; comparing the first and second circuit identification clusters to determine whether the same circuits are present in each of the first and second circuit identification clusters. Typically when the first and second circuit identification clusters comprise the same circuits meaning that a positive signal according to appropriate functioning is triggered. The automatic verification that the circuits corresponding to a particular zone of wetting are correctly activated may thus be attained.

Preferably, the method herein comprises the step of automatically identifying a product characteristic comprising at least one of size, absorbency, and article type. This automatic identification step being carried out after the step of joining the clip-on device to the absorbent article. In this embodiment, each connection track (5) may be seen as an individual bit in a series of bits. For example an article with 7 connection tracks will have a bit string of 7 bits. Each product characteristic may be embedded in the connection track (5) design such that each one, two or more electrical terminals (4) coming into connection with one, two or more connection tracks (5) associated with a product identifier (e.g. a product identifier track) triggers a "1" integer and a no connection a "0" integer. The string of 7 bits may for example be 0010010 associated with a size M Plus, 0100010 with a size M Extra Plus, 1100000 with a size M Super and so on. In this manner a binary coding may be more easily immediately detected by the clip-on device when connected to the article and is able to automatically hence determine to which e.g. article size/absorbency the 7 bit digits correspond to. Further examples and embodiments are described in EP3936102, which may be adopted herein.

Preferably, the predetermined amount of liquid is applied automatically in response to the identified product characteristic. Advantageously this allows to run product-individual (e.g. size absorbency etc.) specific tests to minimize false positives in quality control data.

Preferably a plurality of the sensor-comprising absorbent articles are tested substantially simultaneously. Advantageously this allows operators to acquire a significant amount of quality data during production batches and/or upon receipt of new batches.

## Claims

1. A testing device (1) for testing one or more conductivity parameters of a sensor-comprising absorbent article, said device (1) comprising:
- a computing device (2) comprising a processor and an input receiver and optionally a display;
- a clip-on device (3) comprising a power source, transmitter, a processing unit, and one or more electrical terminals (4) in electrical communication with at least one of the transmitter and the processing unit; wherein said terminals (4) are adapted to come into contact with corresponding connection tracks (5) of a sensor (6), and wherein an absorbent article (7) comprises said sensor (6);
**characterised in that** said computing device (2) and said clip-on device (3) are distinct components in data communication with each other and **in that** when a plurality of conductivity parameters are acquired by the clip-on device (3) and transmitted by the transmitter to the computing device (2), the acquired plurality of conductivity parameters are able to be further processed by the processor and/or processing unit to provide an indication to a user related to the proper functioning of the sensor (6) .

2. A testing device according to Claim 1 wherein the absorbent article (7) is an incontinence article selected from the group consisting of a diaper, a pant, a pad, and combinations thereof.

3. A testing device according to any of the preceding Claims wherein the sensor (6) comprises an electrically conductive material, preferably an electrically conductive ink, having a predetermined pattern.

4. A testing device according to any of the preceding Claims further comprising a test bench (8) comprising a housing (9) for accommodating the absorbent article therein and one or more liquid dispensers (10, 10', 10") arranged to apply a predetermined amount of liquid onto a body facing surface of said absorbent article.

5. A testing device according to Claim 4 comprising a plurality of the liquid dispensers (10, 10', 10") positioned at a distance from one another, preferably such that each dispenser is able to apply a predetermined amount of liquid at one or more of: a different location on the same absorbent article and the same location of a plurality of absorbent articles.

6. A testing device according to Claim 5 wherein each of the plurality of liquid dispensers comprises at least a first dispenser (10) and at least a second dispenser (10'), wherein the at least first dispenser is adapted to apply a first predetermined amount of liquid to a first absorbent article and the at least second dispenser is adapted to apply a second predetermined amount of liquid to a second absorbent article, wherein the at least first and second absorbent articles have a product characteristic that is the same or different and wherein the at least first and second predetermined amounts of liquid are the same or different respectively, wherein said product characteristic comprises at least one of size, absorbency, and article type,.

7. A testing device according to any of Claims 4 to 6 further comprising a liquid reservoir (11) being in fluid communication with the one or more liquid dispensers (10, 10', 10"), preferably via a liquid impermeable tube or pipe (12).

8. A testing device according to any of Claims 4 to 7 further comprising a camera (13) for taking one or more images of the article at different time intervals before, during and after application of liquid by the one or more liquid dispensers (10, 10', 10").

9. A testing device according to any of Claims 4 to 8 wherein the housing comprises a control chamber (14) adapted for receiving the absorbent article to be tested, the chamber being temperature and/or humidity controlled to room conditions at about 21°C and about 50%RH, preferably the chamber being accessible via an opening that may be sealed by a door.

10. A method for testing one or more conductivity parameters of a sensor-comprising absorbent article, said method comprising the steps of:
- providing a testing device (1) comprising:
i. a computing device (2) comprising a processor and an input receiver and optionally a display; and
ii. a clip-on device (3) comprising a power source, transmitter, a processing unit, and one or more electrical terminals (4) in electrical communication with at least one of the transmitter and the processing unit;
- providing an absorbent article (7) comprising a sensor (6) for exudate detection, said sensor comprising a pattern of electrically conductive material and a plurality of connection tracks (5);
- joining the clip-on device to the absorbent article such that at least some of the one or more electrical terminals (4) is in electrical communication corresponding one or more connection tracks (5);
- acquiring one or more analogue conductivity parameters and converting said analogue conductivity parameters to a digital format comprising a binary signal(s);
- transmitting said binary signal(s) from the clip-on device (3) to the computing device (2); and
- processing said signal(s) to automatically provide a status warning to a user in relation to the proper functioning of the sensor (6), wherein the processing step comprises comparing each said signal with a predetermined threshold.

11. A method according to Claim 10 wherein prior to the step of acquiring one or more analogue conductivity parameters, a predetermined amount of liquid is applied to said article and preferably wherein said predetermined amount of liquid is different for each different product characteristic of said article said product characteristic comprising at least one of size, absorbency, and article type, preferably wherein the article type is selected from a pant, a diaper or a pad.

12. A method according to any of Claims 10 to 11 wherein after the step of joining the clip-on device to the absorbent article, a product characteristic comprising at least one of size, absorbency, and article type is automatically identified.

13. A method according to Claims 11 and 12 wherein the predetermined amount of liquid is applied automatically in response to the identified product characteristic.

14. A method according to any of Claims 10 to 13 wherein a plurality of the sensor-comprising absorbent articles are tested substantially simultaneously.

15. A method according to any of Claims 10 to 14 comprising the step of providing a testing device (1) according to any of Claims 1 to 9; or wherein said method is carried out using the testing device (1) according to any of Claims 1 to 9.

## Patentansprüche

1. Prüfvorrichtung (1) zum Prüfen eines oder mehrerer Leitfähigkeitsparameter eines sensorumfassenden absorbierenden Artikels, die Vorrichtung (1) umfassend:
- eine Rechenvorrichtung (2), umfassend einen Prozessor und einen Eingabeempfänger und optional eine Anzeige;
- eine Aufsteckvorrichtung (3), umfassend eine Energiequelle, einen Sender, eine Verarbeitungseinheit und einen oder mehrere elektrische Anschlüsse (4) in elektrischer Kommunikation mit mindestens einem von dem Sender und der Verarbeitungseinheit; wobei die Anschlüsse (4) angepasst sind, um mit entsprechenden Verbindungsbahnen (5) eines Sensors (6) in Kontakt zu kommen, und wobei ein absorbierender Artikel (7) den Sensor (6) umfasst;
**dadurch gekennzeichnet, dass** die Rechenvorrichtung (2) und die Aufsteckvorrichtung (3) unterschiedliche Komponenten in Datenkommunikation miteinander sind, und **dadurch, dass,** wenn eine Vielzahl von Leitfähigkeitsparametern durch die Aufsteckvorrichtung (3) erfasst und durch den Sender an die Rechenvorrichtung (2) gesendet wird, die erfasste Vielzahl von Leitfähigkeitsparametern in der Lage ist, durch den Prozessor und/oder die Verarbeitungseinheit weiterverarbeitet zu werden, um einem Benutzer eine Anzeige bezüglich des ordnungsgemäßen Funktionierens des Sensors (6) bereitzustellen.

2. Prüfvorrichtung nach Anspruch 1, wobei der absorbierende Artikel (7) ein Inkontinenzartikel ist, der aus der Gruppe ausgewählt ist, bestehend aus einer Windel, einer Hose, einer Einlage und Kombinationen davon.

3. Prüfvorrichtung nach einem der vorstehenden Ansprüche, wobei der Sensor (6) ein elektrisch leitfähiges Material umfasst, vorzugsweise eine elektrisch leitfähige Tinte, das ein zuvor bestimmtes Muster aufweist.

4. Prüfvorrichtung nach einem der vorstehenden Ansprüche, ferner umfassend einen Prüfstand (8), umfassend ein Gehäuse (9) zum Unterbringen des absorbierenden Artikels darin und einen oder mehrere Flüssigkeitsspender (10, 10', 10"), die angeordnet sind, um eine zuvor bestimmte Menge an Flüssigkeit auf eine körperzugewandte Oberfläche des absorbierenden Artikels aufzubringen.

5. Prüfvorrichtung nach Anspruch 4, umfassend eine Vielzahl der Flüssigkeitsspender (10, 10', 10"), die in einem Abstand voneinander positioniert sind, vorzugsweise derart, dass jeder Spender in der Lage ist, eine zuvor bestimmte Menge an Flüssigkeit an einer oder mehreren aufzubringen von: einer unterschiedlichen Stelle auf demselben absorbierenden Artikel und derselben Stelle einer Vielzahl von absorbierenden Artikeln.

6. Prüfvorrichtung nach Anspruch 5, wobei jeder der Vielzahl von Flüssigkeitsspendern mindestens einen ersten Spender (10) und mindestens einen zweiten Spender (10') umfasst, wobei der mindestens erste Spender angepasst ist, um eine erste zuvor bestimmte Menge an Flüssigkeit auf einen ersten absorbierenden Artikel aufzubringen, und der mindestens zweite Spender angepasst ist, um eine zweite zuvor bestimmte Menge an Flüssigkeit auf einen zweiten absorbierenden Artikel aufzubringen, wobei der mindestens erste und zweite absorbierende Artikel eine Produkteigenschaft aufweisen, die gleich oder unterschiedlich ist, und wobei die mindestens erste und zweite zuvor bestimmte Menge an Flüssigkeit jeweils gleich oder unterschiedlich sind, wobei die Produkteigenschaft mindestens eines von Größe, Absorptionsvermögen und Artikelart umfasst.

7. Prüfvorrichtung nach einem der Ansprüche 4 bis 6, ferner umfassend ein Flüssigkeitsreservoir (11), das in Fluidkommunikation mit dem einen oder den mehreren Flüssigkeitsspendern (10, 10', 10") steht, vorzugsweise über ein(e) flüssigkeitsundurchlässige(s) Rohr oder Leitung (12).

8. Prüfvorrichtung nach einem der Ansprüche 4 bis 7, ferner umfassend eine Kamera (13) zum Aufnehmen eines oder mehrerer Bilder des Artikels zu unterschiedlichen Zeitintervallen vor, während und nach dem Aufbringen von Flüssigkeit durch den einen oder die mehreren Flüssigkeitsspender (10, 10', 10").

9. Prüfvorrichtung nach einem der Ansprüche 4 bis 8, wobei das Gehäuse eine Steuerkammer (14) umfasst, die zum Empfangen des zu prüfenden absorbierenden Artikels angepasst ist, wobei die Kammer temperatur- und/oder feuchtigkeitsgesteuert auf Raumbedingungen bei etwa 21 °C und etwa 50 % RH ist, vorzugsweise wobei die Kammer über eine Öffnung zugänglich ist, die durch eine Tür verschlossen werden kann.

10. Verfahren zum Prüfen eines oder mehrerer Leitfähigkeitsparameter eines sensorumfassenden absorbierenden Artikels, das Verfahren umfassend die Schritte:
- Bereitstellen einer Prüfvorrichtung (1), umfassend:
i. eine Rechenvorrichtung (2), umfassend einen Prozessor und einen Eingabeempfänger und optional eine Anzeige; und
ii. eine Aufsteckvorrichtung (3), umfassend eine Energiequelle, einen Sender, eine Verarbeitungseinheit und einen oder mehrere elektrische Anschlüsse (4) in elektrischer Kommunikation mit mindestens einem von dem Sender und der Verarbeitungseinheit;
- Bereitstellen eines absorbierenden Artikels (7), umfassend einen Sensor (6), für eine Exsudatdetektion, der Sensor umfassend ein Muster aus elektrisch leitfähigem Material und eine Vielzahl von Verbindungsbahnen (5);
- Zusammenfügen der Aufsteckvorrichtung mit dem absorbierenden Artikel derart, dass mindestens einige des einen oder der mehreren elektrischen Anschlüsse (4) in elektrischer Kommunikation mit einer oder mehreren entsprechenden Verbindungsbahnen (5) stehen;
- Erfassen eines oder mehrerer analoger Leitfähigkeitsparameter und Umwandeln der analogen Leitfähigkeitsparameter in ein digitales Format, umfassend ein oder mehrere binäre Signal(e);
- Senden des/der binären Signals/Signale von der Aufsteckvorrichtung (3) an die Rechenvorrichtung (2); und
- Verarbeiten des/der Signals/Signale, um einem Benutzer automatisch eine Statuswarnung bezüglich des ordnungsgemäßen Funktionierens des Sensors (6) bereitzustellen, wobei der Verarbeitungsschritt ein Vergleichen jedes Signals mit einem zuvor bestimmten Schwellenwert umfasst.

11. Verfahren nach Anspruch 10, wobei vor dem Schritt des Erfassens eines oder mehrerer analoger Leitfähigkeitsparameter eine zuvor bestimmte Menge an Flüssigkeit auf den Artikel aufgebracht wird und vorzugsweise wobei die zuvor bestimmte Menge an Flüssigkeit für jede unterschiedliche Produkteigenschaft des Artikels unterschiedlich ist, die Produkteigenschaft umfassend mindestens eines von Größe, Absorptionsvermögen und Artikelart, vorzugsweise wobei die Artikelart aus einer Hose, einer Windel oder einer Einlage ausgewählt ist.

12. Verfahren nach einem der Ansprüche 10 bis 11, wobei nach dem Schritt des Zusammenfügens der Aufsteckvorrichtung mit dem Absorptionsartikel eine Produkteigenschaft, umfassend mindestens eines von Größe, Absorptionsfähigkeit und Artikelart, automatisch identifiziert wird.

13. Verfahren nach den Ansprüchen 11 und 12, wobei die zuvor bestimmte Menge an Flüssigkeit als Reaktion auf die identifizierte Produkteigenschaft automatisch aufgebracht wird.

14. Verfahren nach einem der Ansprüche 10 bis 13, wobei eine Vielzahl der sensorumfassenden absorbierenden Artikel im Wesentlichen gleichzeitig geprüft wird.

15. Verfahren nach einem der Ansprüche 10 bis 14, umfassend den Schritt des Bereitstellens einer Prüfvorrichtung (1) nach einem der Ansprüche 1 bis 9; oder wobei das Verfahren unter Verwendung der Prüfvorrichtung (1) nach einem der Ansprüche 1 bis 9 vorgenommen wird.

## Revendications

1. Dispositif de test (1) permettant de tester un ou des paramètres de conductivité d'un article absorbant comprenant un capteur, ledit dispositif (1) comprenant :
- un dispositif informatique (2) comprenant un processeur et un récepteur d'entrée et facultativement un affichage ;
- un dispositif à clipser (3) comprenant une source de puissance, un transmetteur, une unité de traitement, et une ou des bornes électriques (4) en communication électrique avec au moins l'un parmi le transmetteur et l'unité de traitement ; dans lequel lesdites bornes (4) sont conçues pour venir en contact avec des tracés de connexion (5) correspondants d'un capteur (6), et dans lequel un article absorbant (7) comprend ledit capteur (6) ;
**caractérisé en ce que** ledit dispositif informatique (2) et ledit dispositif à clipser (3) sont des composants distincts en communication de données l'un avec l'autre et **en ce que** lorsqu'une pluralité de paramètres de conductivité sont acquis par le dispositif à clipser (3) et transmis par le transmetteur au dispositif informatique (2), la pluralité acquise de paramètres de conductivité sont aptes à être ultérieurement traités par le processeur et/ou l'unité de traitement pour fournir une indication à un utilisateur concernant le fonctionnement adéquat du capteur (6).

2. Dispositif de test selon la revendication 1 dans lequel l'article absorbant (7) est un article d'incontinence choisi dans le groupe constitué d'une couche, d'une culotte, d'une serviette, et de combinaisons de celles-ci.

3. Dispositif de test selon l'une quelconque des revendications précédentes dans lequel le capteur (6) comprend un matériau électroconducteur, de préférence une encre électroconductrice, ayant un motif prédéterminé.

4. Dispositif de test selon l'une quelconque des revendications précédentes comprenant en outre un banc de test (8) comprenant un logement (9) permettant d'accueillir l'article absorbant à l'intérieur de celui-ci et un ou des distributeurs de liquide (10, 10', 10") agencés pour appliquer une quantité prédéterminée de liquide sur une surface faisant face vers le corps, dudit article absorbant.

5. Dispositif de test selon la revendication 4 comprenant une pluralité de distributeurs de liquide (10, 10', 10") positionnés à une distance les uns des autres, de préférence de telle sorte que chaque distributeur est apte à appliquer une quantité prédéterminée de liquide au niveau d'une ou plusieurs parmi : une localisation différente sur le même article absorbant et la même localisation d'une pluralité d'articles absorbants.

6. Dispositif de test selon la revendication 5 dans lequel chacun parmi la pluralité de distributeurs de liquide comprend au moins un premier distributeur (10) et au moins un second distributeur (10'), dans lequel l'au moins un premier distributeur est conçu pour appliquer une première quantité prédéterminée de liquide sur un premier article absorbant et l'au moins un second distributeur est conçu pour appliquer une seconde quantité prédéterminée de liquide sur un second article absorbant, dans lequel au moins les premier et second articles absorbants ont une caractéristique de produit qui est identique ou différente et dans lequel au moins les première et seconde quantités prédéterminées de liquide sont identiques ou différentes respectivement, dans lequel ladite caractéristique de produit comprend au moins l'un parmi la taille, l'absorbance et le type d'article.

7. Dispositif de test selon l'une quelconque des revendications 4 à 6 comprenant en outre un réservoir de liquide (11) étant en communication fluidique avec le ou les distributeurs de liquide (10, 10', 10"), de préférence par l'intermédiaire d'un tube ou tuyau (12) imperméable aux liquides.

8. Dispositif de test selon l'une quelconque des revendications 4 à 7 comprenant en outre une caméra (13) permettant de prendre une ou des images de l'article à différents intervalles de temps avant, pendant et après application de liquide par le ou les distributeurs de liquide (10, 10', 10").

9. Dispositif de test selon l'une quelconque des revendications 4 à 8 dans lequel le logement comprend une chambre de régulation (14) conçue pour recevoir l'article absorbant à tester, la chambre étant régulée en température et/ou en humidité à des conditions ambiantes à environ 21 °C et environ 50 % HR, de préférence la chambre étant accessible par l'intermédiaire d'une ouverture qui peut être fermée par une porte.

10. Procédé permettant de tester un ou des paramètres de conductivité d'un article absorbant comprenant un capteur, ledit procédé comprenant les étapes consistant à :
- fournir un dispositif de test (1) comprenant :
i. un dispositif informatique (2) comprenant un processeur et un récepteur d'entrée et facultativement un affichage ; et
ii. un dispositif à clipser (3) comprenant une source de puissance, un transmetteur, une unité de traitement, et une ou des bornes électriques (4) en communication électrique avec au moins l'un parmi le transmetteur et l'unité de traitement ;
- fournir un article absorbant (7) comprenant un capteur (6) pour détection d'exsudats, ledit capteur comprenant un motif de matériau électroconducteur et une pluralité de tracés de connexion (5) ;
- joindre le dispositif à clipser à l'article absorbant de telle sorte qu'au moins certaines parmi la ou les bornes (4) électriques sont en communication électrique correspondant à un ou des tracés de connexion (5) ;
- acquérir un ou des paramètres de conductivité analogiques et convertir lesdits paramètres de conductivité analogiques en un format numérique comprenant un ou des signaux binaires ;
- transmettre ledit ou lesdits signaux binaires à partir du dispositif à clipser (3) vers le dispositif informatique (2) ; et
- traiter ledit ou lesdits signaux pour fournir automatiquement un avertissement de statut à un utilisateur par rapport au fonctionnement adéquat du capteur (6), dans lequel l'étape de traitement comprend la comparaison de chaque signal précité à un seuil prédéterminé.

11. Procédé selon la revendication 10 dans lequel avant l'étape d'acquisition d'un ou des paramètres de conductivité analogiques, une quantité prédéterminée de liquide est appliquée audit article et de préférence dans lequel ladite quantité prédéterminée de liquide est différente pour chaque caractéristique de produit différente dudit article, ladite caractéristique de produit comprenant au moins l'un parmi la taille, l'absorbance et le type d'article, de préférence dans lequel le type d'article est choisi parmi une culotte, une couche ou une serviette.

12. Procédé selon l'une quelconque des revendications 10 à 11 dans lequel après l'étape consistant à joindre le dispositif à clipser à l'article absorbant, une caractéristique de produit comprenant au moins l'un parmi la taille, l'absorbance et le type d'article est automatiquement identifiée.

13. Procédé selon les revendications 11 et 12 dans lequel la quantité prédéterminée de liquide est appliquée automatiquement en réponse à la caractéristique de produit identifiée.

14. Procédé selon l'une quelconque des revendications 10 à 13 dans lequel une pluralité des articles absorbants comprenant un capteur sont testés sensiblement en même temps.

15. Procédé selon l'une quelconque des revendications 10 à 14 comprenant l'étape de fourniture d'un dispositif de test (1) selon l'une quelconque des revendications 1 à 9 ; ou dans lequel ledit procédé est effectué à l'aide du dispositif de test (1) selon l'une quelconque des revendications 1 à 9.
